# FASCICULE DE BREVET EUROPEEN

(11) **EP 2 905 034 B1**
(45) Date de publication et mention de la délivrance du brevet: **01.05.2024**
(21) Numéro de dépôt: 14154133.4
(22) Date de dépôt: 06.02.2014
(51) Int. Cl.: A61L 2/18, A61L 2/238, A61Q 11/02

(54) **SOLUTION D'IRRIGATION À NANOPARTICULES POUR UTILISATION DANS UNE MÉTHODE DE TRAITEMENT ENDODONTIQUE.**
IRRIGATIONSLÖSUNG MIT NANOPARTIKELN ZUR VERWENDUNG IN EINER ENDODONTISCHEN BEHANDLUNG
IRRIGATION SOLUTION WITH NANOPARTICLES FOR USE IN A METHOD OF ENDODONTIC TREATMENT

(43) Date de publication de la demande: 12.08.2015
(73) Titulaire: Maillefer Instruments Holding S.À.R.L., 1338 Ballaigues (CH)
(72) Inventeur: Le Ouay, Benjamin, 1024 Ecublens (CH); Stellacci, Francesco, 1110 Morges (CH); Conde, Janine, 1400 Yverdon-les-Bains (CH); Mefti, Selma, 1003 Lausanne (CH)
(74) Mandataire: Micheli & Cie SA

(56) Documents cités:
- DE-U1-202008 009 873
- US-A1- 2007 053 850
- US-A1- 2009 047 222
- US-A1- 2010 316 685
- US-A1- 2011 262 556
- US-A1- 2012 021 034
- US-A1- 2013 115 248
- US-B2- 6 924 116

## Description

### DOMAINE DE L'INVENTION ET ETAT DE LA TECHNIQUE

La présente invention concerne une solution d'irrigation pour utilisation dans une méthode de traitement endodontique.

Lors d'un traitement endodontique, le dentiste utilise une solution d'irrigation pour nettoyer et désinfecter le canal radiculaire après chaque passage de lime. En effet, la phase d'instrumentation du canal radiculaire avec des limes mécanisées ou non crée une grande quantité de débris qui, avec les tissus organiques, constituent la boue dentinaire. Cette fine couche qui obstrue les tubules dentinaires doit être enlevée avant l'étape finale d'obturation du canal. L'état de l'art actuel préconise un rinçage systématique après chaque passage de lime avec de l'hypochlorite de sodium (NaOCl) suivi d'un nettoyage final à l'EDTA (acide éthylènediaminetétraacétique), ces deux solutions d'irrigation ne pouvant pas être utilisées simultanément car l'une annule l'action de l'autre.

Dans la demande de brevet australien AU 2012100480 A4 est proposée une autre solution d'irrigation, comprenant de l'éthanol, de la soude (hydroxyde de sodium) et des nanoparticules antibactériennes, telles que des nanoparticules d'argent, de zinc ou d'or.

Les nanoparticules d'argent sont en effet connues dans le domaine médical pour leurs propriétés antibactériennes. L'effet antibactérien des nanoparticules d'argent semble se produire lors de leur dissolution et de la libération d'ions Ag⁺. En général, l'oxydant permettant la dissolution des nanoparticules d'argent est le dioxygène dissous dans l'eau.

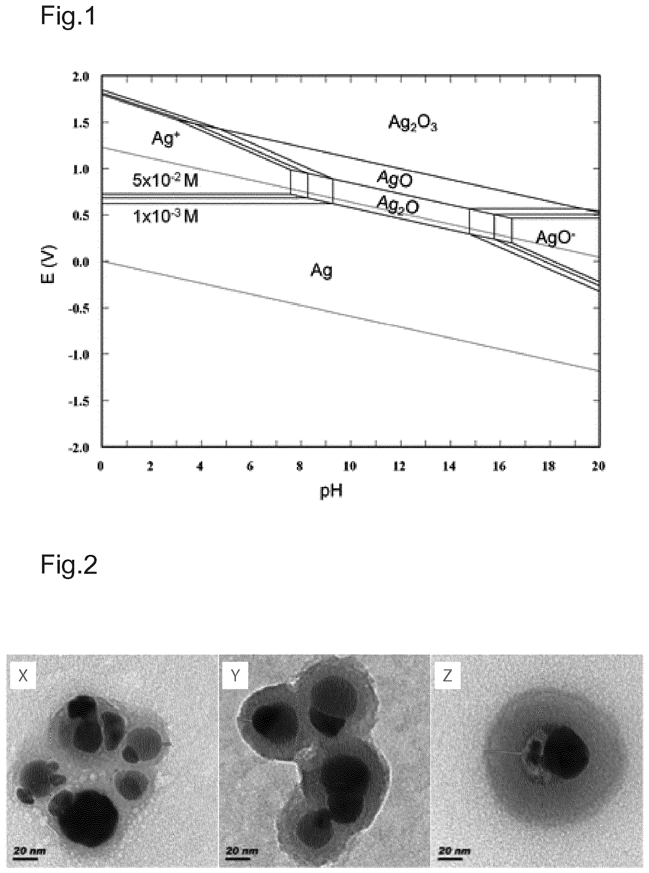

La solution d'irrigation proposée dans la demande de brevet australien précitée est basée sur ce principe de libération d'ions d'argent. Toutefois il n'apparaît pas clairement comment la dissolution des nanoparticules dans la solution est obtenue. La présence d'hydroxyde de sodium dans la formulation, et le pH basique qui en résulte, favorisent la formation d'oxyde d'argent, ce qui constitue même un frein à l'oxydation de ces nanoparticules et donc à leur dissolution, comme le montre le diagramme de la figure 1 ci-annexée. Il en résulte que l'action des nanoparticules pendant le traitement endodontique, et probablement même après, est faible.

La demande de brevet américain US2011/262556 décrit une composition comprenant du peroxyde d'hydrogène et des nanoparticules d'argent. Le peroxyde d'hydrogène permet de fragiliser la paroi cellulaire des bactéries et promeut l'entrée des particules d'argent antibactériennes dans les bactéries pour les détruire.

Le modèle d'utilité allemand DE 20 2008 009 873 U1 décrit une composition de blanchiment dentaire comprenant des nanoparticules de dioxyde de titane TiO₂ photoactives dopées au carbone ainsi qu'un agent oxydant tel que le peroxyde d'hydrogène. Les nanoparticules de TiO₂ sont dopées pour pouvoir être activables non seulement par un rayonnement UV mais également par la lumière visible.

La demande de brevet américain US2013/115248 décrit une composition pour le nettoyage des implants et/ou le débridement des surfaces dures dans la cavité buccale. Cette composition comprend du peroxyde d'hydrogène, des nanoparticules de TiO₂ et des microparticules solides.

La demande de brevet américain US2009/0047222 décrit un dentifrice comprenant des nanoparticules encapsulées dans des coques en silice et des composés peroxydés. L'objectif de cette demande de brevet est de proposer une composition de dentifrice ou de bain de bouche colorée comprenant des nanoparticules colorées, des composés peroxydés et des ions de fluorure et dont la coloration perdure.

Le brevet américain US 6,924,116 B2 décrit un procédé de production de nanoparticules encapsulées dans des coques de silice et fonctionnalisées par des groupements chimiques particuliers.

La demande de brevet américain US 2010/316685 décrit un mélange d'hydrosol d'argent et de peroxyde d'hydrogène aqueux pour des applications endodontiques, chacun de ces agents ayant une action désinfectante qui lui est propre.

La demande de brevet américain US 2007/053850 propose des compositions désinfectantes dont l'innocuité a été améliorée. Ces compositions utilisent des nanoparticules à base d'alliage comme agent bactéricide.

La demande de brevet américain US 2012/021034 décrit des nanoparticules encapsulées dans des coquilles de silice mésoporeuses ayant une activité antimicrobienne.

### RESUME DE L'INVENTION

La présente invention vise à proposer une solution d'irrigation à nanoparticules pour utilisation dans une méthode de traitement endodontique, dont la durée de vie des nanoparticules puisse être ajustée pour par exemple :
- correspondre à la durée d'un traitement endodontique, soit environ 30 à 60 minutes, pour une action désinfectante forte pendant tout ledit traitement, ou
- être supérieure à la durée d'un traitement endodontique, afin que certaines particules partiellement oxydées puissent résider dans le canal radiculaire et exercer une action bactéricide forte dans les heures, voire les jours, qui suivent le traitement.

A cette fin il est prévu selon l'invention un ensemble d'une première et d'une deuxième préparation destinées à être mélangées avant ou pendant un traitement endodontique pour former une solution d'irrigation endodontique, la première préparation comprenant un agent oxydant, la deuxième préparation comprenant des nanoparticules antibactériennes traitées pour ralentir leur oxydation par l'agent oxydant.

Ainsi, dans la présente invention il est possible de jouer sur les deux paramètres que sont la quantité d'agent oxydant et la protection conférée par le traitement des nanoparticules antibactériennes pour ajuster de manière assez précise la durée de vie des nanoparticules, c'est-à-dire leur vitesse de dissolution. L'oxydation des nanoparticules antibactériennes commence après le mélange des première et deuxième préparations, entraînant une libération progressive d'ions à action antibactérienne. Les deux paramètres précités peuvent être choisis pour obtenir un fort effet désinfectant pendant tout le traitement endodontique et/ou un effet à plus long terme.

Dans le cadre de la présente invention, les nanoparticules antibactériennes sont encapsulées dans des coquilles et forment ainsi avec ces dernières des structures hybrides coeur-coquille.

Les nanoparticules antibactériennes sont en argent.

Les coquilles sont mésoporeuses. Elles sont par exemple en silice, en oxyde de titane, en oxyde de zirconium ou en polymère.

Avantageusement, les coquilles comprennent à leur surface des groupements fonctionnels greffés.

L'agent oxydant est par exemple un peroxyde, un hypochlorite, un halogène, un permanganate, un perchlorate ou un periodate.

Dans un mode de réalisation particulier, l'agent oxydant est le peroxyde d'hydrogène.

La première préparation peut comprendre en outre un agent chélateur, par exemple l'acide maléique, l'acide citrique, l'acide éthylènediaminetétraacétique, l'acide malique, l'acide gluconique, l'acide lactique, l'acide glycolique, l'acide propanoïque, l'acide acétique, l'acide malonique, l'acide oxalique, l'acide tartrique, l'acide phosphorique, un sel des précédents acides cités, ou l'éthylènediamine.

La deuxième préparation peut comprendre en outre un agent tensioactif, tel qu'un sulfate, un sulfonate, un phophate, un carboxylate d'alkyle, d'aryle-alkyle ou d'éther-alkyle, un ammonium quaternaire, un polysorbate ou un polymère di- ou tri-bloc. En particulier, l'agent tensioactif peut être le nitrate de cetyltrimethylammonium.

De préférence, les première et deuxième préparations sont telles que leur mélange présente un pH acide.

Avantageusement, la composition de la première préparation est choisie pour que la première préparation ait une action nettoyante sur la dentine indépendamment de son action oxydante sur les nanoparticules antibactériennes.

### BREVE DESCRIPTION DES DESSINS

- La figure 1 est un diagramme potentiel-pH (dit « de Pourbaix ») calculé pour l'argent.
- La figure 2 représente des nanoparticules d'argent encapsulées dans des coquilles de silice d'épaisseur variable (X : 8 nm ; Y : 15 nm ; Z : 30 nm).

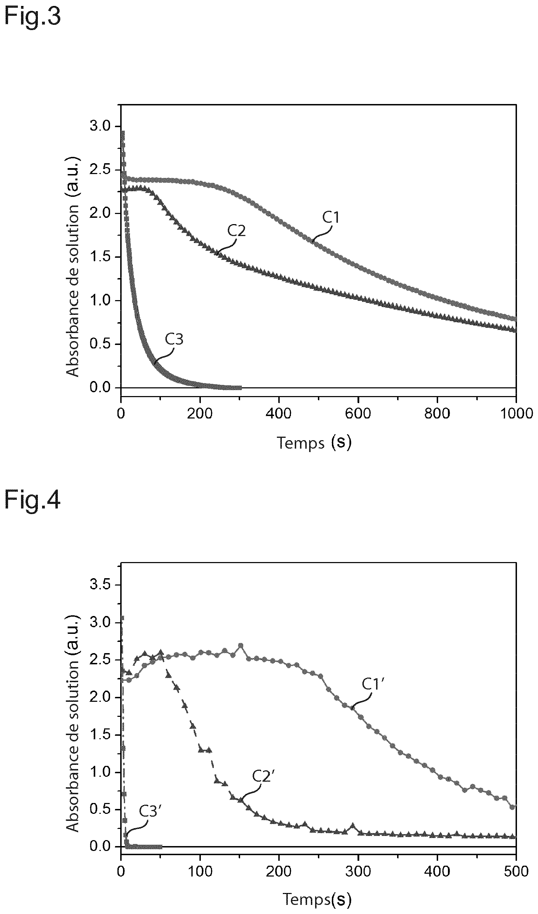

- La figure 3 est un diagramme représentant l'évolution de l'absorbance de solutions d'irrigation en fonction du temps. Sur cette figure, C1 désigne la courbe d'absorbance d'une solution d'irrigation selon l'invention contenant des nanoparticules d'argent encapsulées dans des coquilles de silice d'épaisseur 30 nm, C2 désigne la courbe d'absorbance d'une solution d'irrigation selon l'invention contenant des nanoparticules d'argent encapsulées dans des coquilles de silice d'épaisseur 15 nm, et C3 désigne la courbe d'absorbance d'une solution d'irrigation similaire à celles des courbes C1 et C2 mais dont les nanoparticules d'argent sont nues, c'est-à-dire non encapsulées dans des coquilles.
- La figure 4 est un diagramme représentant l'évolution de l'absorbance d'autres solutions d'irrigation en fonction du temps. Sur cette figure, C1' désigne la courbe d'absorbance d'une solution d'irrigation selon une variante de l'invention contenant des nanoparticules d'argent encapsulées dans des coquilles de silice d'épaisseur 30 nm, C2' désigne la courbe d'absorbance d'une solution d'irrigation selon ladite variante de l'invention contenant des nanoparticules d'argent encapsulées dans des coquilles de silice d'épaisseur 15 nm, et C3' désigne la courbe d'absorbance d'une solution d'irrigation similaire à celles des courbes C1' et C2' mais dont les nanoparticules d'argent sont nues.
- La figure 5 représente de la boue dentinaire recouvrant la paroi d'un canal radiculaire après instrumentation de ce dernier sans irrigation.
- La figure 6 représente la paroi d'un canal radiculaire après instrumentation de ce dernier avec irrigation au moyen d'une solution d'irrigation selon l'invention.

### DESCRIPTION DÉTAILLÉE DE PLUSIEURS MODES DE RÉALISATION

La solution d'irrigation endodontique selon l'invention est obtenue en mélangeant deux préparations A et B, se présentant de préférence sous la forme de solutions liquides, de préférence aqueuses. La solution A contient un agent oxydant et un agent chélateur. La solution B contient des nanoparticules d'argent hybrides à structure coeur-coquille et un agent tensioactif. Par « nanoparticules » on entend dans le cadre de la présente invention des particules ayant un diamètre inférieur à 1000 nm, typiquement compris entre 5 et 1000 nm. De manière connue en soi, les nanoparticules hybrides à structure coeur-coquille comprennent des nanoparticules dites de « coeur » encapsulées dans des coquilles. En fonction des conditions de synthèse, il est possible qu'une ou plusieurs nanoparticules de coeur soient encapsulées dans une même coquille, comme le montre la figure 2. Cette même figure 2 montre que l'épaisseur des coquilles est variable. Selon les conditions de formation des nanoparticules, l'épaisseur des coquilles peut être plus ou moins grande de manière à contrôler la vitesse de dissolution des particules de coeur, comme cela sera expliqué plus loin.

Selon un mode de réalisation préféré de l'invention, l'agent oxydant est du peroxyde d'hydrogène (ou eau oxygénée), l'agent chélateur est de l'acide maléique, les nanoparticules hybrides comprennent des nanoparticules de coeur en argent et des nanoparticules de coquille en silice (Ag@SiO₂), et l'agent tensioactif est du CTAN (nitrate de cetyltrimethylammonium).

Lorsque les solutions A et B sont mélangées, le peroxyde d'hydrogène (H₂O₂) oxyde l'argent métallique Ag⁰ des nanoparticules pour libérer des ions argent Ag⁺, cette espèce étant stable du fait du pH acide de la solution d'irrigation A+B. La coquille de silice formée autour des nanoparticules d'argent protège ces dernières et ralentit la libération des ions argent qui, sinon, seraient libérés en quelques dizaines de secondes seulement. La combinaison d'un agent oxydant, le peroxyde d'hydrogène, et d'une coquille de protection autour des nanoparticules d'argent permet de régler la cinétique de dissolution de l'argent pour que cette dissolution se produise par exemple sur une durée correspondant à la durée d'un traitement endodontique, typiquement 30 à 60 minutes environ, ou sur une durée plus longue. Par son action de chélation, l'acide maléique aide à dissoudre les nanoparticules d'argent une fois qu'elles ont été oxydées, en les passivant et en empêchant la formation de Ag₂O. Enfin, le CTAN disperse les nanoparticules d'argent et favorise donc aussi leur dissolution.

En plus de leur fonction sus indiquée, le peroxyde d'hydrogène et l'acide maléique exercent une action de nettoyage de la dentine et d'élimination de la boue dentinaire lors de l'irrigation du canal radiculaire par la solution d'irrigation selon l'invention. Plus précisément, le milieu acide et oxydant permet la dissolution des résidus inorganiques (notamment l'hydroxyapatite) et organiques (notamment les tissus nécrotiques et les biofilms bactériens). Par ailleurs, le CTAN rend la silice mésoporeuse et participe aussi au nettoyage du canal radiculaire par son action détergente. La présence de nanoparticules dans la solution d'irrigation permet également d'obtenir une action abrasive, qui augmente l'efficacité de l'élimination de la boue dentinaire.

La solution d'irrigation endodontique proposée possède donc deux actions distinctes :
- la solution A+B permet ainsi un nettoyage immédiat reposant sur le caractère oxydant, acide, chélatant, détergent et abrasif de ses différents composants ;
- la solution A permet d'activer la dissolution des nanoparticules d'argent, ce qui contribue à leur action bactéricide. La présence de la coquille de silice autour de celles-ci permet d'en contrôler la vitesse de dissolution, pour la rendre compatible avec la durée d'un traitement endodontique. La figure 3 montre la diminution progressive de l'absorbance de la solution d'irrigation A+B (cf. courbe C1 : solution dans laquelle les nanoparticules Ag@SiO₂ ont une épaisseur de coquille SiO₂ de 30 nm ; courbe C2 : solution dans laquelle les nanoparticules Ag@SiO₂ ont une épaisseur de coquille SiO₂ de 15 nm), et donc la dissolution progressive des nanoparticules d'argent. A titre de comparaison, on peut voir sur la figure 3 que la dissolution de nanoparticules d'argent sans coquille est très rapide et incompatible avec la durée d'un traitement endodontique (cf. courbe C3).

Ainsi, une particularité de la présente invention réside dans le fait que les agents permettant le nettoyage immédiat du canal radiculaire sont aussi ceux qui permettent la dissolution des nanoparticules d'argent.

Dans la présente invention telle que décrite ci-dessus, la dissolution progressive des nanoparticules d'argent est possible grâce au fait que l'on se place en milieu acide-oxydant, où l'ion Ag⁺ est stable en solution (cf. figure 1). Il est néanmoins possible de considérer d'autres moyens de stabiliser les ions argent, par exemple par la présence d'une haute concentration en ions Cl⁻ ou bien par la présence d'ammoniaque ou d'amines. La figure 4 représente des courbes d'absorbance C1', C2', C3' dans une telle variante de l'invention, où la solution A comprend du peroxyde d'hydrogène et, à la place de l'acide maléique, du nitrate d'ammonium. Le pH de la solution est alors voisin de 7, et les espèces de l'argent (+I) sont stables en solution du fait de la formation de complexes Ag(NH₃)ₙ⁺. Sur cette figure 4 on peut noter que le point d'inflexion marquant le début de la dissolution effective se situe à la même durée après mélange des solutions A et B (50 s pour une coquille de 15 nm d'épaisseur, et 250 s pour une coquille de 30 nm) que dans le cas de la figure 3. La vitesse de dissolution est ensuite plus élevée, ce mélange étant plus efficace pour dissoudre les nanoparticules. Ceci témoigne du rôle de la coquille de silice autour des nanoparticules d'argent pour le ralentissement de leur dissolution, et ce indépendamment de la solution choisie pour les dissoudre.

En plus de limiter la vitesse de dissolution des nanoparticules antibactériennes, la coquille de silice peut servir d'interface pour le greffage de groupements fonctionnels. En effet, le greffage de groupements sur une nanoparticule antibactérienne d'argent nue implique de fixer les groupes directement sur les atomes de surface de la nanoparticule, par exemple au moyen de fonctions thiols. La présence d'une telle fonctionnalisation peut modifier énormément la réactivité des nanoparticules, et donc notamment leur vitesse de dissolution. De plus, à mesure que la dissolution des nanoparticules se produit, les groupements greffés se retrouveraient détachés de la surface de la nanoparticule, et perdraient ainsi leur action. Pour ces raisons, il est préférable de réaliser autour de la nanoparticule antibactérienne une couche d'interface, sur laquelle il est possible de greffer les groupements fonctionnels, et qui soit relativement stable au cours de la dissolution de la nanoparticule.

La silice constitue ainsi un matériau idéal pour réaliser cette couche d'interface, en raison de sa stabilité dans les conditions de la solution d'irrigation et de la variété d'organosilanes facilement disponibles, ce qui permet un vaste choix de fonctionnalisation. D'autres matériaux et moyens de greffage peuvent néanmoins être envisagés. Une telle fonctionnalisation a pour but de modifier les propriétés physiques ou chimiques de surface des nanoparticules, afin de modifier leur comportement.

Les groupes greffés peuvent être :
- de petits groupements fonctionnels (sulfonate, ammonium, etc.) servant à modifier la charge de surface des nanoparticules et ainsi leur affinité pour des surfaces de charge opposée, comme la surface de la dentine ;
- de petits groupements fonctionnels (alkyl, phényl, alcool, etc.) servant à modifier la nature hydrophobe/hydrophile des nanoparticules ;
- de petits groupements fonctionnels (carboxylates, phosphonates, thiols, amines, etc.) servant à donner aux nanoparticules une affinité plus élevée pour un type de surface particulier ;
- des chaînes polymériques (par exemple le polyéthylène glycol), qui peuvent s'enchevêtrer avec les biopolymères, et augmenter ainsi préférentiellement l'adhésion sur les biofilms bactériens (mécanisme de muco-adhésion) ;
- de petites molécules spécifiques, susceptibles d'interagir avec des récepteurs moléculaires donnés, et d'immobiliser ainsi les nanoparticules au voisinage de ces récepteurs ;
- des peptides ou des protéines, qui pourraient se lier à des objets spécifiques (par exemple dans la membrane des bactéries) et permettre ainsi l'adressage précis de nanoparticules antibactériennes.

Typiquement, dans la solution A le peroxyde d'hydrogène est présent à hauteur de 0,1 à 30% en masse, de préférence à hauteur d'environ 6%, et l'acide maléique est présent à hauteur de 1 à 60% en masse, de préférence à hauteur d'environ 12%.

Dans la solution B, qui peut avoir le même volume que la solution A, la quantité de nanoparticules hybrides Ag@SiO₂ est typiquement comprise entre 10 et 10000 mg/L, et de préférence égale à environ 400 mg/L (environ 300 mg/L d'argent et environ 100 mg/L de silice), et la proportion en masse de CTAN est typiquement comprise entre 0,1 et 2,5%, et de préférence égale à environ 0,5%. Le diamètre des nanoparticules d'argent est typiquement compris entre 5 et 250 nm, et de préférence égal à environ 30 nm. L'épaisseur des coquilles de silice est typiquement comprise entre 2 et 100 nm.

En pratique, la solution A et la solution B peuvent être contenues dans deux récipients séparés, mis à la disposition du dentiste sous la forme d'un kit. La solution A et la solution B peuvent aussi être contenues dans deux compartiments séparés d'un même récipient. La solution A est stable dans le temps. La solution B l'est aussi, à condition qu'elle soit stockée sans contact avec l'oxygène.

Avant ou pendant le traitement endodontique le dentiste mélange les deux solutions A et B pour former la solution d'irrigation, avec laquelle il irriguera le canal radiculaire après chaque instrumentation de ce dernier. Le mélange des solutions A et B peut aussi être fait dans le tuyau du système d'injection. Mélanger les deux solutions A et B met les nanoparticules en contact avec l'agent oxydant et active ainsi l'effet antibactérien de l'argent, effet antibactérien qui subsiste pendant tout le traitement endodontique du fait des coquilles de silice qui ralentissent la dissolution des nanoparticules d'argent. Contrairement aux ions argent, les nanoparticules d'argent et de silice ont une bonne affinité avec la surface des dents et se collent facilement à celle-ci. A la fin du traitement endodontique le canal radiculaire est obturé. Une partie des nanoparticules d'argent, partiellement oxydées, pourront rester dans le canal radiculaire ainsi obturé et continuer leur action désinfectante pendant plusieurs jours après l'opération. Ceci permet d'obtenir une action désinfectante, y compris dans les parties du canal non-directement instrumentées, par libération et diffusion des ions argent.

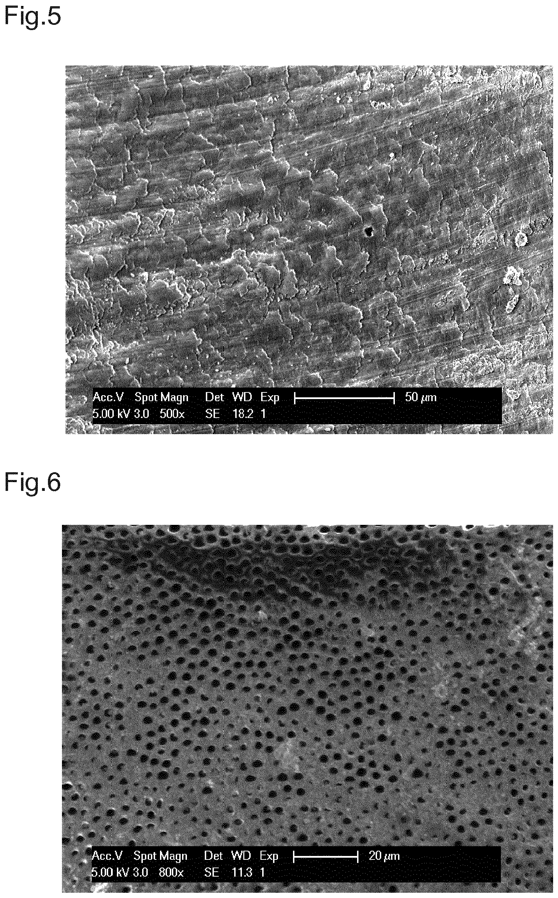

A titre d'illustration, la figure 5 montre la boue dentinaire qui recouvre la paroi du canal radiculaire après instrumentation dudit canal sans irrigation. La figure 6 montre la paroi d'un canal radiculaire après instrumentation dudit canal avec irrigation au moyen de la solution A+B selon l'invention. On peut constater que la boue dentinaire a été enlevée et que les tubules dentinaires sont ouverts et non érodés. La solution d'irrigation selon l'invention, utilisée après chaque passage de lime et aussi comme liquide de rinçage final, permet de nettoyer efficacement la surface des canaux radiculaires. Les nanoparticules d'argent de la solution B peuvent être obtenues d'une manière connue en soi, par exemple à partir de nitrate d'argent et de citrate de sodium.

Les coquilles de silice peuvent être obtenues à partir d'APTES (3-aminopropyltriethoxysilane) et de TEOS (tetraethylorthosilicate). Le TEOS forme la plus grande partie de la matrice de silice. L'APTES aide à lier la silice à la surface de l'argent (par la formation d'une liaison Ag-NH₂R). L'épaisseur des coquilles de silice peut être ajustée, typiquement entre 2 et 100 nm, en ajustant la quantité de TEOS et d'APTES. Il est possible d'avoir plusieurs tailles de coquilles de silice (en préparant plusieurs lots et en les mélangeant) de façon à contrôler la cinétique de dissolution et « l'aplatir ». Ainsi, une partie des nanoparticules d'argent, recouvertes d'une couche de silice moins épaisse, auront un effet désinfectant à plus court terme et une autre partie des nanoparticules d'argent, recouvertes d'une couche de silice plus épaisse, auront un effet à plus long terme.

D'autres additifs peuvent être utilisés pour la synthèse des particules hybrides Ag@SiO₂. Par exemple, le glycérol permet d'obtenir une meilleure monodispersité des particules de coeur en argent.

Dans des variantes de l'invention, d'autres agents oxydants pourraient être utilisés en remplacement ou en plus du peroxyde d'hydrogène, par exemple un autre peroxyde, un hypochlorite, un dihalogène, un permanganate, un perchlorate et/ou un periodate. De même, d'autres agents chélateurs pourraient être utilisés en remplacement ou en plus de l'acide maléique, par exemple l'acide citrique, l'acide éthylènediaminetétraacétique, l'acide malique, l'acide gluconique, l'acide lactique, l'acide glycolique, l'acide propanoïque, l'acide acétique, l'acide malonique, l'acide oxalique, l'acide tartrique, l'acide phosphorique, un sel des précédents acides cités, ou l'éthylènediamine. Des ions halogénures (I⁻, Br⁻, F⁻) peuvent aussi être utilisés pour s'insérer dans la couche d'oxyde et favoriser la dissolution des nanoparticules d'argent. Enfin, le CTAB (bromure de cetyltrimethylammonium) pourrait remplacer ou être ajouté au CTAN. Toutefois le CTAN est préféré au CTAB car l'ion bromure formerait un précipité AgBr très peu soluble en présence d'argent, ce qui diminuerait la disponibilité de celui-ci. De manière générale l'agent tensioactif peut être anionique (tel qu'un sulfate, un sulfonate, un phophate, un carboxylate d'alkyle, d'aryle-alkyle ou d'éther-alkyle), cationique (tel qu'un ammonium quaternaire), zwitterionique ou non-ionique (tel qu'un polysorbate ou un polymère di- ou tri-bloc).

Comme expliqué précédemment, les nanoparticules d'argent sont encapsulées dans des nanoparticules de silice selon une structure coeur-coquille pour ralentir leur oxydation par l'agent oxydant. Comme matériau de coquille on pourrait utiliser un autre matériau que la silice, par exemple l'oxyde de titane, l'oxyde de zirconium ou un revêtement polymère.

Enfin, au lieu d'être sous la forme d'une solution liquide, l'une au moins des préparations A et B pourrait être solide, par exemple sous la forme d'une poudre. Si les deux préparations A et B sont solides, on peut envisager que le dentiste les dissolve lui-même dans de l'eau ou un autre liquide.

## Revendications

1. Ensemble d'une première et d'une deuxième préparation pour une utilisation dans une méthode de traitement endodontique, la première préparation comprenant un agent oxydant, la deuxième préparation comprenant des nanoparticules antibactériennes en argent encapsulées dans des coquilles mésoporeuses pour ralentir leur oxydation par l'agent oxydant, lesdites première et deuxième préparations étant destinées à être mélangées avant ou pendant ledit traitement endodontique pour former une solution d'irrigation endodontique et activer l'effet antibactérien des nanoparticules en permettant à l'agent oxydant d'oxyder les nanoparticules.

2. Ensemble pour utilisation selon la revendication 1, dans lequel les coquilles sont en silice, en oxyde de titane, en oxyde de zirconium ou en polymère.

3. Ensemble pour utilisation selon la revendication 1 ou 2, dans lequel les coquilles comprennent à leur surface des groupements fonctionnels greffés.

4. Ensemble pour utilisation selon l'une quelconque des revendications 1 à 3, dans lequel l'agent oxydant est un peroxyde, un hypochlorite, un halogène, un permanganate, un perchlorate ou un periodate.

5. Ensemble pour utilisation selon la revendication 4, dans lequel l'agent oxydant est le peroxyde d'hydrogène.

6. Ensemble pour utilisation selon l'une quelconque des revendications 1 à 5, dans lequel la première préparation comprend en outre un agent chélateur.

7. Ensemble pour utilisation selon la revendication 6, dans lequel l'agent chélateur est l'acide maléique, l'acide citrique, l'acide éthylènediaminetétraacétique, l'acide malique, l'acide gluconique, l'acide lactique, l'acide glycolique, l'acide propanoïque, l'acide acétique, l'acide malonique, l'acide oxalique, l'acide tartrique, l'acide phosphorique, un sel des précédents acides cités, ou l'éthylènediamine.

8. Ensemble pour utilisation selon l'une quelconque des revendications 1 à 7, dans lequel la deuxième préparation comprend en outre un agent tensioactif.

9. Ensemble pour utilisation selon la revendication 8, dans lequel l'agent tensioactif est un sulfate, un sulfonate, un phophate, un carboxylate d'alkyle, d'aryle-alkyle ou d'éther-alkyle, un ammonium quaternaire, un polysorbate ou un polymère di- ou tri-bloc.

10. Ensemble pour utilisation selon la revendication 8 ou 9, dans lequel l'agent tensioactif est le nitrate de cetyltrimethylammonium.

11. Ensemble pour utilisation selon l'une quelconque des revendications 1 à 10, dans lequel les première et deuxième préparations sont telles que leur mélange présente un pH acide.

## Patentansprüche

1. Menge eines ersten und eines zweiten Präparats für eine Verwendung in einem endontischen Behandlungsverfahren, wobei das erste Präparat ein Oxidationsmittel umfasst, das zweite Präparat antibakterielle Nanopartikel aus Silber umfasst, die in mesoporösen Schalen eingekapselt sind, um ihre Oxidation durch das Oxidationsmittel zu verlangsamen, wobei das erste und das zweite Präparat dazu bestimmt sind, vor oder während der endontischen Behandlung gemischt zu werden, um eine endontische Irrigationslösung zu bilden und die antibakterielle Wirkung der Nanopartikel zu aktivieren, indem sie es dem Oxidationsmittel erlauben, die Nanopartikel zu oxidieren.

2. Menge zur Verwendung nach Anspruch 1, wobei die Schalen aus Silizium, aus Titanoxid, aus Zirkonoxid oder aus Polymer bestehen.

3. Menge zur Verwendung nach Anspruch 1 oder 2, wobei die Schalen an ihrer Oberfläche gepfropfte Funktionsgruppen umfassen.

4. Menge zur Verwendung nach einem der Ansprüche 1 bis 3, wobei das Oxidationsmittel ein Peroxid, ein Hypochlorit, ein Halogen, ein Permanganat, ein Perchlorat oder ein Periodat ist.

5. Menge zur Verwendung nach Anspruch 4, wobei das Oxidationsmittel Wasserstoffperoxid ist.

6. Menge zur Verwendung nach einem der Ansprüche 1 bis 5, wobei das erste Präparat ferner einen Chelatbildner umfasst.

7. Menge zur Verwendung nach Anspruch 6, wobei der Chelatbildner Maleinsäure, Zitronensäure, Ethylendiamintetraessigsäure, Maleinsäure, Gluconsäure, Milchsäure, Glykolsäure, Propionsäure, Essigsäure, Malonsäure, Oxalsäure, Weinsäure, Phosphorsäure, ein Salz der vorhergehend genannten Säuren oder Ethylendiamin ist.

8. Menge zur Verwendung nach einem der Ansprüche 1 bis 7, wobei das zweite Präparat ferner ein Tensid umfasst.

9. Menge zur Verwendung nach Anspruch 8, wobei das Tensid ein Sulfat, ein Sulfonat, ein Phosphat, ein Alkyl-, Aryl-Alkyl- oder Etheralkyl-Carboxylat, ein quaternäres Ammonium, ein Polysorbat oder ein Di- oder Tri-Block-Polymer ist.

10. Menge zur Verwendung nach Anspruch 8 oder 9, wobei das Tensid Cetyltrimethylammoniumnitrat ist.

11. Menge zur Verwendung nach einem der Ansprüche 1 bis 10, wobei das erste und das zweite Präparat derart sind, dass ihr Gemisch einen sauren pH-Wert aufweist.

## Claims

1. Set of a first and a second preparation for use in an endodontic treatment method, the first preparation comprising an oxidising agent, the second preparation comprising silver antibacterial nanoparticles encapsulated in mesoporous shells to slow their oxidation by the oxidising agent, said first and second preparations being intended to be mixed before or during said endodontic treatment to form an endodontic irrigation solution and to activate the antibacterial effect of the nanoparticles by allowing the oxidising agent to oxidise the nanoparticles.

2. Set for use as claimed in claim 1, wherein the shells are made of silica, titanium oxide, zirconium oxide or polymer.

3. Set for use as claimed in claim 1 or 2, wherein the shells comprise grafted functional groups on their surface.

4. Set for use as claimed in any one of claims 1 to 3, wherein the oxidising agent is a peroxide, a hypochlorite, a halogen, a permanganate, a perchlorate or a periodate.

5. Set for use as claimed in claim 4, wherein the oxidising agent is hydrogen peroxide.

6. Set for use as claimed in any one of claims 1 to 5, wherein the first preparation further comprises a chelating agent.

7. Set for use as claimed in claim 6, wherein the chelating agent is maleic acid, citric acid, ethylene diamine tetraacetic acid, malic acid, gluconic acid, lactic acid, glycolic acid, propanoic acid, acetic acid, malonic acid, oxalic acid, tartaric acid, phosphoric acid, a salt of the acids cited above, or ethylene diamine.

8. Set for use as claimed in any one of claims 1 to 7, wherein the second preparation further comprises a surfactant.

9. Set for use as claimed in claim 8, wherein the surfactant is a sulfate, a sulfonate, a phosphate, an alkyl, alkyl aryl or alkyl ether carboxylate, a quaternary ammonium, a polysorbate or a di- or tri-block polymer.

10. Set for use as claimed in claim 8 or 9, wherein the surfactant is cetyl trimethylammonium nitrate.

11. Set for use as claimed in any one of claims 1 to 10, wherein the first and second preparations are such that the mixture thereof has an acid pH.
